# EUROPEAN PATENT SPECIFICATION

(11) **EP 3 874 029 B1**
(45) Date of publication and mention of the grant of the patent: **30.08.2023**
(21) Application number: 19878837.4
(22) Date of filing: 29.10.2019
(51) Int. Cl.: C12N 5/10, A61K 35/12, A61K 38/18, A61K 38/46, A61K 48/00, A61P 37/00, C07K 14/475, C07K 14/51, C12N 15/18, C12N 15/55, C12N 15/85, C12N 5/0775, C12N 9/18, A61K 35/28, C07K 14/515

(54) **GENETICALLY-ENGINEERED MESENCHYMAL STEM CELLS OVEREXPRESSING AOAH AND USES THEREOF**
GENETISCH VERÄNDERTE MESENCHYMALE STAMMZELLEN, DIE AOAH ÜBEREXPRIMIEREN, UND DEREN VERWENDUNGEN
CELLULES SOUCHES MÉSENCHYMATEUSES GÉNÉTIQUEMENT MODIFIÉES SUREXPRIMANT AOAH ET UTILISATIONS CORRESPONDANTES

(30) Priority: 29.10.2018 US 201862752000 P
(43) Date of publication of application: 08.09.2021
(73) Proprietor: Ottawa Hospital Research Institute, Ottawa, Ontario K1H 8L6 (CA); United Therapeutics Corporation, Silver Spring, MD 20910 (US)
(72) Inventor: CALLAHAN, Michael, Boulder, Colorado 80301 (US); MEI, Shirley H.J., Ottawa, Ontario K1G 2L4 (CA); WANG, Yan, Ottawa, Ontario K1G 3T4 (CA); FLORIAN, Maria, Québec H9B 2J1 (CA); ROWE, Katelynn, Ottawa, Ontario K2J 0A3 (CA); LEE, Chyan-jang, Ottawa, Ontario K1T 0T8 (CA)
(74) Representative: Lavoix
(86) International application number: PCT/CA2019/051526
(87) International publication number: WO 2020/087160

(56) References cited:
- WO-A1-2016/001846
- WO-A2-2010/015929
- LU MINGFANG ET AL: "Stimulus-dependent Deacylation of Bacterial Lipopolysaccharide by Dendritic Cells", JOURNAL OF EXPERIMENTAL MEDICINE, vol. 197, no. 12, 16 June 2003 (2003-06-16), pages 1745-1754, XP055935101, US ISSN: 0022-1007, DOI: 10.1084/jem.20030420 Retrieved from the Internet: URL:https://www.ncbi.nlm.nih.gov/pmc/artic les/PMC2193946/pdf/20030420.pdf>
- SHAO BAOMEI ET AL: "A Host Lipase Detoxifies Bacterial Lipopolysaccharides in the Liver and Spleen", JOURNAL OF BIOLOGICAL CHEMISTRY, vol. 282, no. 18, 1 May 2007 (2007-05-01), pages 13726-13735, XP055935096, US ISSN: 0021-9258, DOI: 10.1074/jbc.M609462200
- ZAKI, O. S. et al.: "Bone-Marrow Mesenchymal Stem Cells Combat Lipopolysaccharide-Induced Sepsis in Rats via Amendment of P38-MAPK Signaling Cascade", Inflammation, vol. 41, April 2018 (2018-04), pages 541-554, XP036468398, ISSN: 1573-2576
- OJOGUN, J. et al.: "Overproduction of Acyloxyacyl Hydrolase by Macrophages and Dendritic Cells Prevents Prolonged Reactions to Bacterial Lipopolysaccharide In Vivo", The Journal of Infectious Diseases, vol. 200, 2009, pages 1685-93, XP055706704, ISSN: 1537-6613
- PORADA, C. D. et al.: "Mesenchymal Stem Cells as Therapeutics as Vehicles for Gene and Drug Delivery", Adv. Drug Deliv. Rev., vol. 62, 2010, pages 1156-66, XP027509884, ISSN: 1872-8294, DOI: 10.1016/j.addr.2010.08.010
- WEIL, B. R. et al.: "The Immunomodulatory Properties of Mesenchymal Stem Cells: Implications for Surgical Diseases", J. Surg. Res, vol. 167, 2011, pages 78-86, XP028156548, ISSN: 1095-8673, DOI: 10.1016/j.jss.2010.07.019
- NOVOTNY, N. M. et al.: "Angiopoietin-1 in the Treatment of Ischemia and Sepsis", Shock, vol. 31, 2009, pages 335-341, XP055706705, ISSN: 1540-0514
- OU, H. et al.: "Comparison of Bone Marrow Tissue-and Adipose Tissue-derived Mesenchymal Stem Cells in Treatment of Sepsis in a Murine Model of Lipopolysaccharide-induced Sepsis", Molecular Medicine Reports, vol. 14, 2016, pages 3862-70, XP055706711, ISSN: 1791-3004

## Description

### TECHNICAL FIELD

The present disclosure generally relates to the treatment of inflammatory diseases such as systemic inflammatory response syndrome (SIRS), multiple organ dysfunction symptom (MODS), septic shock and sepsis.

### BACKGROUND ART

Systemic inflammatory response syndrome (SIRS) is a serious condition related to systemic inflammation that results in multiple organ dysfunctions; acute lung injury (ALI) represents the most common and earliest organ failure. SIRS may occur as a consequence of various pathogenic events, with sepsis syndrome being the most prevalent and lethal cause.

Sepsis annually affects over a million people in the United States with a risk of death from sepsis being as high as 30%, severe sepsis as high as 50%, and septic shock as high as 80%. Treatment for sepsis is listed as the most expensive condition treated in U.S. hospitals, costing more than $20 billion in 2011 (Torio et al., Healthcare Cost and Utilization Project (HCUP) Statistical Brief, #160, 2013, 1-12). With more than 258,000 lives being lost yearly, sepsis ranks as the third leading cause of death in the U.S. after heart disease and cancer. Subsequently, survivors often face long-term effects post-sepsis, including amputations, anxiety, memory loss, chronic pain and fatigue, and worsened cognitive function.

Sepsis results from the overproduction of inflammatory mediators as a consequence of the interaction of the immune system with bacteria and bacterial wall constituents. Lipopolysaccharide (LPS), also known as endotoxin and the major outer membrane component of Gram-negative bacteria, is one of the causative agents known to trigger an adverse immune response.

Dissemination of bacteria in the blood results in a cytokine storm, which converts a healthy immune system, which normally functions to control blood-borne infections, into a liability for the patient.

Despite the use of antibiotics as a control strategy for bacterial sepsis, the rise in antibiotic resistance by bloodstream bacterial isolates in recent years has highlighted an urgent need for novel compounds, therapies and regimes for the treatment and management of sepsis, its symptoms and pathologies.

WO2016/001846 discloses mesenchymal stem cells (MSCs) for use in the treatment of sepsis.

Lu et al. discloses that acyloxyacyl hydrolase (AOAH) produced by dendritic cells can detoxify LPS.

Novotny et al. discloses that MSCs recombinantly expressing angiopoietin-1 (ANGPT1) improve the survival rate of animal models of sepsis.

Objects, advantages and features of the present disclosure will become apparent upon reading of the following description of specific embodiments thereof, given by way of example only with reference to the accompanying drawings.

### BRIEF DESCRIPTION OF DRAWINGS

In the appended drawings:
**FIG. 1A** depicts the construction of the plasmid (#2) comprising the AOAH coding sequence under the control of an EF-1 promoter.
**FIG. 1B** depicts the construction of the plasmid (#3) comprising the AOAH coding sequence under the control of a CMV promoter.
**FIG. 1C** depicts the construction of the plasmid (#1) comprising the AOAH coding sequence under the control of a CMV promoter and a CMV enhancer.
**FIG. 2** is a graph showing the expression of AOAH by MSC transfected with plasmid #1 (black bar) (ii) plasmid #2 (dark grey bar) and (iii) plasmid #3 (light grey bar). Expression was measured by Western Blot on cell lysates and the expression was normalized based on betaactin expression.
**FIGs. 3A** and **3B** are graphs showing the total internal AOAH expression (**FIG. 3A**) and the proportion of mature AOAH relative to total AOAH expression (**FIG. 3B**) in MSC transfected with the plasmid #1 and harvested 24h, 48h and 72h post-transfection. Expression was measured by Western Blot on cell lysates and the expression was normalized based on betaactin expression.
**FIG. 3C** is a graph showing the levels of AOAH in the culture medium of MSC transfected with the plasmid #1 and harvested 24h, 48h and 72h post-transfection. Expression was measured by ELISA.
**FIG. 4A** is a schematic of the endotoxin-induced sepsis animal model used in the present studies. Endotoxemia was induced in mice by intraperitoneal injection of LPS, and the mice were treated with AOAH-transfected MSC (NT002), untransfected MSC or vehicle 1h post-LPS injection. At 20h post-LPS injection, plasma from mice was harvested to determine the plasmatic levels of pro-inflammatory cytokines or chemokines.
**FIGs. 4B-4I** are graphs showing the levels of the pro-inflammatory cytokines or chemokines IL-6 (**FIG. 4B**), IL-10 (**FIG. 4C**), IL-12 (**FIG. 4D**), MIP-1a (**FIG. 4E**), eotaxin (**FIG. 4F**), G-CSF (**FIG. 4G**), KC (**FIG. 4H**) and MCP-1 (FIG. 4I) at 20h post-LPS injection in the plasma of mice treated with NT002 (n = 8), untransfected MSC (n = 8) or vehicle (n = 7). * P < 0.05; ** P < 0.01 (One-way ANOVA test).
**FIG. 5A** is a schematic of the *E. coli*-induced sepsis animal model used in the present studies. *E*. *coli* was induced in mice by intraperitoneal injection, and the mice were treated with NT002 (or vehicle) 1h post-bacteria injection. Animal wellness was assessed for 5 days to determine the survival rate.
**FIG. 5B** shows the survival rate of mice infected with *E*. *coli* and treated with NT002 or vehicle. Sham mice (no sepsis) received vehicle treatment as control. * P < 0.05 (Log-rank test).
**FIG. 6A** depicts the construction of plasmid #4 comprising the AOAH coding sequence and ANGPT1 coding sequence.
**FIG. 6B** shows the codon-optimized sequence of the nucleic acid encoding human AOAH used in the studies described herein (SEQ ID NO: 1). The coding sequence is in bold, the first 6 nucleotides represent the Kozak sequence and the last three nucleotides correspond to the stop codon.
**FIG. 6C** shows the sequence of the nucleic acid encoding human ANGPT1 used in the studies described herein (SEQ ID NO: 3). The coding sequence is in bold, the first 6 nucleotides represent the Kozak sequence and the last three nucleotides correspond to the stop codon.
**FIG. 6D** shows the survival rate of mice infected with *E*. *coli* and treated with NT003 (AOAH/ANGPT1-transfected MSC) or vehicle.
**FIG. 7A** is a schematic of the cecal ligation and puncture (CLP)-induced sepsis animal model used in the present studies. Once sepsis was induced, the mice were treated with NT003 (AOAH/ANGPT1-transfected MSCs) or vehicle 6h post-CLP. Sham mice (no sepsis) received vehicle treatment as control. Animal wellness was assessed for 5 days to determine the survival rate.
**FIG. 7B** shows the combined survival data of CLP mice treated with NT003, or vehicle.
**FIG. 8A** shows the amino acid sequence of human AOAH (isoform 1, UniProtKB accession number P28039, SEQ ID NO:5), with the signal peptide sequence underlined (residues 1-23), the propeptide sequence (residues 24-34) in italics and the sequence of the mature form (residues 35-575) in bold.
**FIG. 8B** shows the nucleotide sequence of the cDNA encoding human AOAH (isoform 1, NCBI Reference Sequence: NM_001637.3, SEQ ID NO:6), with the coding sequence in bold.
**FIG. 9A** shows the amino acid sequence of human ANGPT1 (isoform 1, UniProtKB accession number Q15389-1, SEQ ID NO:7), with the signal peptide sequence underlined (residues 1-15) and the sequence of the mature form (residues 16-498) in bold.
**FIGs. 9B** and **9C** show the nucleotide sequence of the cDNA encoding human ANGPT1 (isoform 1, NCBI Reference Sequence: NM_001146.4, SEQ ID NO:8), with the coding sequence in bold.

### DETAILED DISCLOSURE

The use of the terms "a" and "an" and "the" and similar referents in the context of describing the technology (especially in the context of the following claims) are to be construed to cover both the singular and the plural, unless otherwise indicated herein or clearly contradicted by context.

The terms "comprising", "having", "including", and "containing" are to be construed as open-ended terms (i.e., meaning "including, but not limited to") unless otherwise noted.

All methods described herein can be performed in any suitable order unless otherwise indicated herein or otherwise clearly contradicted by context.

The use of any and all examples, or exemplary language ("*e.g*.", "such as") provided herein, is intended merely to better illustrate embodiments of the claimed technology and does not pose a limitation on the scope unless otherwise claimed.

No language in the specification should be construed as indicating any non-claimed element as essential to the practice of embodiments of the claimed technology.

Herein, the term "about" has its ordinary meaning. The term "about" is used to indicate that a value includes an inherent variation of error for the device or the method being employed to determine the value, or encompass values close to the recited values, for example within 10% of the recited values (or range of values).

Recitation of ranges of values herein are merely intended to serve as a shorthand method of referring individually to each separate value falling within the range, unless otherwise indicated herein, and each separate value is incorporated into the specification as if it were individually recited herein. All subsets of values within the ranges are also incorporated into the specification as if they were individually recited herein.

Where features or aspects of the disclosure are described in terms of Markush groups or list of alternatives, those skilled in the art will recognize that the disclosure is also thereby described in terms of any individual member, or subgroup of members, of the Markush group or list of alternatives.

Unless specifically defined otherwise, all technical and scientific terms used herein shall be taken to have the same meaning as commonly understood by one of ordinary skill in the art (e.g., in stem cell biology, cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T. A. Brown (editor), Essential Molecular Biology: A Practical Approach, Volumes 1 and 2, IRL Press (1991), D. M. Glover and B. D. Hames (editors), DNA Cloning: A Practical Approach, Volumes 1-4, IRL Press (1995 and 1996), and F. M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J. E. Coligan et al. (editors) Current Protocols in Immunology, John Wiley & Sons (including all updates until present).

In the studies described herein, the preparation of mesenchymal stem cells (MSCs) that are genetically modified to overexpress certain proteins of interest is described. More specifically, MSCs genetically modified to overexpress an acyloxyacyl hydrolase (AOAH) polypeptide, as well as both AOAH and angiopoeitin-1 (ANGPT1), were shown to reduce inflammation and improve survival in murine models of SIRS/sepsis. The results provide evidence that MSCs genetically modified to overexpress AOAH alone or in combination with ANGPT1 may be useful for the prevention or treatment of SIRS/sepsis in humans.

In a first aspect, the present disclosure relates to a mesenchymal stem cell (MSC) that is genetically modified to overexpress an acyloxyacyl hydrolase (AOAH) polypeptide.

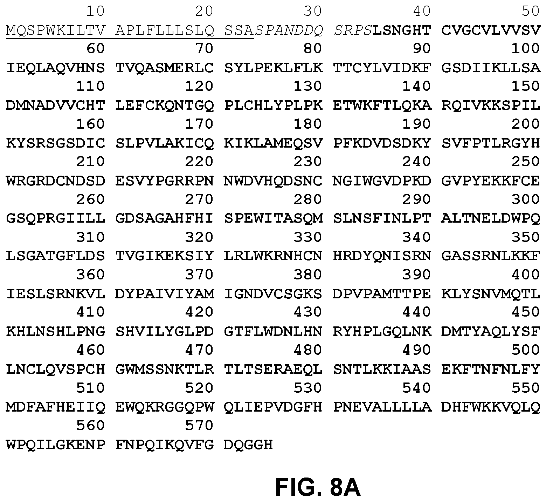

Acyloxyacyl hydrolase (AOAH, EC 3.1.1.77) is a protein of 575 amino acids in human (UniProtKB accession number P28039, **FIG. 8A**, SEQ ID NO:5) that includes an amino-terminal signal peptide of 23 amino acids as well as a propeptide of 11 amino acids (residues 24-34). It comprises two disulfide-linked subunits: a small subunit encompassing residues 35-156 and a large subunit encompassing residues 157-575. AOAH is mainly produced by monocytemacrophages, neutrophils, dendritic cells, and renal cortical epithelial cells, and selectively removes the secondary (acyloxyacyl-linked) fatty acyl from lipid A present in Gram-negative bacterial lipopolysaccharides (LPS). Its active site is the serine at position 263 located in the Gly-Asp-Ser-(Leu) [GDS(L)] motif. Mutations of residues 61, 173, 345 and 379 have been shown to have no effect on enzymatic activity, whereas enzymatic activity is decreased or lost by mutations at residues 263, 372 and 419.

In an embodiment, the MSC is further genetically modified to overexpress a second polypeptide, preferably a polypeptide that may be useful for the treatment of SIRS/sepsis. In an embodiment, the second polypeptide is an angiopoetin-1 (ANGPT1) polypeptide.

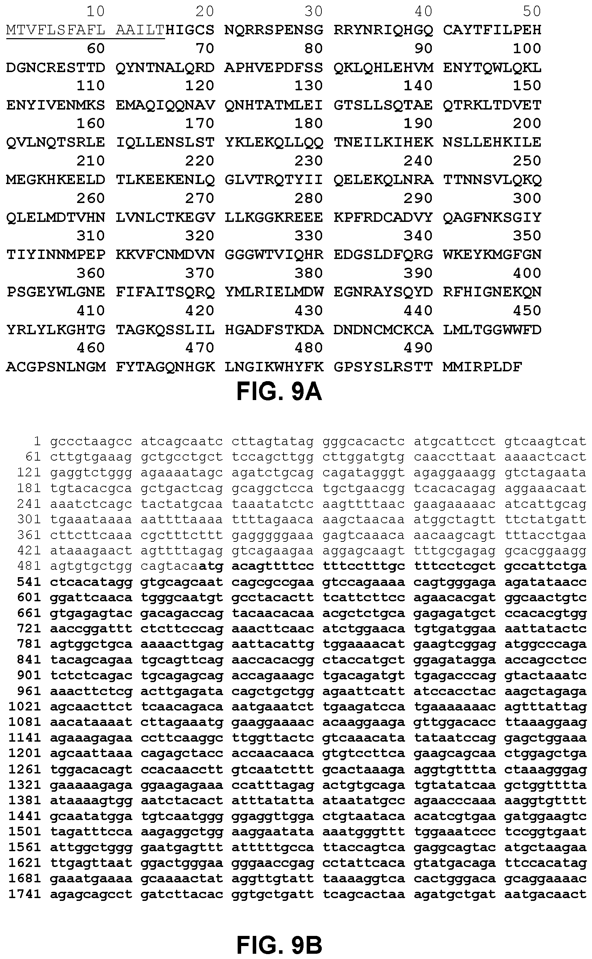

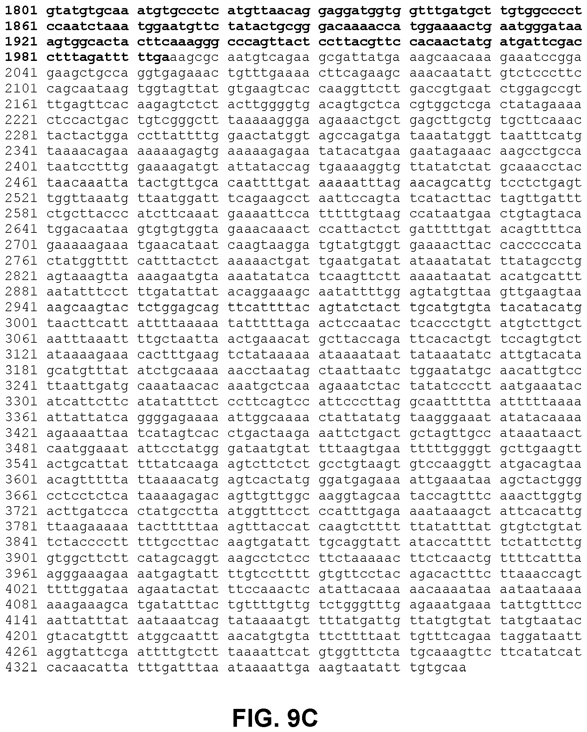

Angiopoietin-1 (ANGPT1) is a protein of 498 amino acids in human (UniProtKB accession number Q15389, **FIG. 9A**, SEQ ID NO:7) that includes an amino-terminal signal peptide of 15 amino acids. It comprises a super clustering domain encompassing residues 81-119 and a central coiled domain encompassing residues 153-261 that are involved in ANGPT1 oligomerization, and a Fibrinogen C-terminal domain encompassing residues 277-497 that is involved in the binding to its target receptor. This secreted glycoprotein binds and activates the TEK/TIE2 receptor tyrosine kinase (CD202B), expressed almost exclusively in endothelial cells, by inducing its dimerization and tyrosine phosphorylation. Mutation of position 119 (A119S) has been shown to reduce binding capability to its receptor. Mutation of position 249 (K249R) has been detected in subjects with primary congenital glaucoma (PCG) (associated with a defect in ANGPT/TEK signaling) and is believed to reduce its biological activity. Deletion of the five C-terminal amino acids (R494*) is predicted to destabilize the protein (Thomson et al., J Clin Invest. 2017 Dec 1; 127(12): 4421-4436).

The term "overexpress" as used herein means that the level of expression of AOAH and/or of the second polypeptide (*e.g*., ANGPT1) in the genetically-modified MSC is higher than the level of expression of AOAH and/or of the second polypeptide (*e.g*., ANGPT1) in a corresponding MSC that does not comprise the genetic modification(s). In an embodiment, the level of expression of AOAH and/or of the second polypeptide (*e.g*., ANGPT1) in the genetically-modified MSC is at least 20%, 50%, 100% (2-fold), 200% (3-fold), 300% (4-fold), 400% (5-fold), 900% (10-fold), 20-fold, 50-fold or 100-fold higher than the level of expression of AOAH and/or of the second polypeptide (*e.g*., ANGPT1) in a corresponding MSC that does not comprise the genetic modification.

In an embodiment, the overexpression of the AOAH protein and/or of the second polypeptide (*e.g*., ANGPT1) is achieved by increasing the expression of the endogenous AOAH gene and/or of the gene encoding the second polypeptide (*e.g*., ANGPT1) in the MSC. This may be achieved, for example, by introducing suitable promoter(s) and/or transcriptional regulatory (*e.g*., enhancer) sequences in transcriptional relationship with (i.e. operably-linked to) the endogenous AOAH gene and/or of the gene encoding the second polypeptide (*e.g*., ANGPT1). Alternatively, this may be achieved by excising/inactivating a transcriptional repressor sequence that inhibits the expression of the endogenous AOAH gene and/or of the gene encoding the second polypeptide (*e.g*., ANGPT1). Introduction of promoter(s) and/or transcriptional regulatory (*e.g*., enhancer) sequences and excision/inactivation of transcriptional repressor sequences to induce overexpression of endogenous AOAH gene in the MSC may be achieved using genome editing techniques. Genome editing techniques can modify gene expression in a target cell by inserting, replacing, or removing DNA in the genome using an artificially engineered nuclease. Examples of such nucleases may include zinc finger nucleases (ZPNs) (Gommans et al., J. Mol Biol, 354(3): 507-519 (2005)), transcription activator-like effector nucleases (TALENs) (Zhang et al., Nature Biotechnol, 29: 149-153 (2011)), the CRISPR/Cas system (Cheng et al., Cell Res., 23: 1163-71] (2013)), and engineered meganucleases (Riviere et al., Gene Ther., 21(5): 529-32 (2014)). The nucleases create specific double-stranded breaks (DSBs) at targeted locations in the genome, and use endogenous mechanisms in the cell to repair the induced break by homologous recombination (HR) and nonhomologous end-joining (NHEJ). Such techniques may be used to achieve overexpression of endogenous AOAH and/or of the endogenous gene encoding the second polypeptide (*e.g*., ANGPT1) in the MSCs.

A first nucleic acid sequence is "operably-linked" with a second nucleic acid sequence when the first nucleic acid sequence is placed in a functional relationship with the second nucleic acid sequence. For instance, a promoter is operably-linked to a coding sequence if the promoter affects the transcription or expression of the coding sequence. Generally, operably-linked DNA sequences are contiguous and, where necessary to join two protein coding regions, in reading frame. However, since for example enhancers generally function when separated from the promoters by several kilobases and intronic sequences may be of variable lengths, some nucleic acids may be operably-linked but not contiguous. "Transcriptional regulatory sequences" or "transcriptional regulatory elements" are generic terms that refer to DNA sequences, such as initiation and termination signals, enhancers, and promoters, splicing signals, polyadenylation signals, etc., which induce or control transcription of protein coding sequences with which they are operably-linked.

In another embodiment, the overexpression of the AOAH protein and/or of the second polypeptide (*e.g*., ANGPT1) is achieved by inducing the expression of a recombinant (i.e. exogenous) AOAH polypeptide and/or of a recombinant second polypeptide (*e.g*., ANGPT1) in the MSC. Thus, in another aspect, the present disclosure relates to a genetically-modified MSC comprising or overexpressing a recombinant (i.e. exogenous) AOAH polypeptide. In an embodiment, the genetically-modified MSC further comprises or overexpresses a recombinant (i.e. exogenous) second polypeptide, preferably ANGPT1.

The term "recombinant" means that something has been recombined, so that when made in reference to a nucleic acid, the term refers to a molecule that is comprised of nucleic acid sequences that are joined together or produced by means of molecular biological techniques. The term "recombinant" when made in reference to a protein or a polypeptide refers to a protein or polypeptide molecule that is not synthesized from the native genome of the cell, e.g., which is expressed from a recombinant nucleic acid construct created by means of molecular biological techniques (whether or not this synthetic nucleic acid construct is integrated in the genome of the cell). Recombinant nucleic acid constructs may include a nucleotide sequence which is ligated to, or is manipulated to become ligated to, a nucleic acid sequence to which it is not ligated in nature, or to which it is ligated at a different location in nature. Referring to a nucleic acid construct as "recombinant" therefore indicates that the nucleic acid molecule has been manipulated using genetic engineering, *i.e*. by human intervention. Recombinant nucleic acid constructs may for example be introduced into a host cell by transformation (*e.g*., transduction or transfection). Such recombinant nucleic acid constructs may include sequences derived from the same host cell species or from different host cell species, which have been isolated and reintroduced into cells of the host species. Recombinant nucleic acid construct sequences may become integrated into a host cell genome, either as a result of the original transformation of the host cells, or as the result of subsequent recombination and/or repair events.

In an embodiment, the recombinant (i.e. exogenous) AOAH polypeptide comprises or consists of a sequence that is at least 50, 60 or 70% identical to the amino acid sequence of mature native human AOAH depicted in **FIG. 8A**/SEQ ID NO:5 (residues 35-575) and that exhibits the enzymatic activity of native human AOAH. In embodiments, the recombinant (i.e. exogenous) AOAH polypeptide comprises or consists of a sequence that is at least 75%, 80%, 85%, 90%, 95%, 96%, 97%, 98% or 99% identical to the amino acid sequence of mature native human AOAH depicted in **FIG. 8A**/SEQ ID NO:5 (residues 35-575) and that exhibits the enzymatic activity of native human AOAH. In an embodiment, the recombinant (i.e. exogenous) AOAH polypeptide comprises or consists of a sequence that is at least 50, 60 or 70% identical to the amino acid sequence of native human AOAH precursor depicted in **FIG. 8A**/SEQ ID NO:5 (residues 24-575) and that exhibits the enzymatic activity of native human AOAH. In embodiments, the recombinant (i.e. exogenous) AOAH polypeptide comprises or consists of a sequence that is at least 75%, 80%, 85%, 90%, 95%, 96%, 97%, 98% or 99% identical to the amino acid sequence of native human AOAH precursor depicted in **FIG. 8A**/SEQ ID NO:5 (residues 24-575) and that exhibits the enzymatic activity of native human AOAH. In an embodiment, the recombinant (i.e. exogenous) AOAH polypeptide comprises or consists of a sequence that is at least 50, 60 or 70% identical to the amino acid sequence of full-length native human AOAH depicted in **FIG. 8A**/SEQ ID NO:5 (residues 1-575) and that exhibits the enzymatic activity of native human AOAH. In embodiments, the recombinant (i.e. exogenous) AOAH polypeptide comprises or consists of a sequence that is at least 75%, 80%, 85%, 90%, 95%, 96%, 97%, 98% or 99% identical to the amino acid sequence of full-length native human AOAH depicted in **FIG.** 8A/SEQ ID NO:5 (residues 1-575) and that exhibits the enzymatic activity of native human AOAH.

Thus, the recombinant (i.e. exogenous) AOAH polypeptide may be the native human AOAH protein (full-length, precursor or mature form) or an enzymatically active variant thereof comprising amino acid deletions, substitutions and/or additions that do not significantly interfere with the enzymatic activity. In an embodiment, the variant exhibits an enzymatic activity that is at least 70%, 75%, 80%, 85%, 90%, 95% or 100% that of native human AOAH. In an embodiment, the variant does not comprise a mutation in the Gly-Asp-Ser-(Leu) [GDS(L)] motif. In an embodiment, the variant does not comprise a mutation at a position corresponding to residues 263, 372 or 419 of full-length native human AOAH. In an embodiment, the variant comprises one or more conservative substitutions. Conservative amino acid substitutions are known in the art, and include amino acid substitutions in which one amino acid having certain physical and or chemical properties is exchanged for another amino acid that has the same chemical or physical properties. For instance, the conservative amino acid substitution can be an acidic amino acid substituted for another acidic amino acid (e.g., Asp to Glu or vice-versa), an amino acid with a nonpolar side chain substituted for another amino acid with a nonpolar side chain (e.g., Ala, Gly, Val, Ile, Leu, Met, Phe, Pro, Trp, Val, etc), a basic amino acid substituted for another basic amino acid (Lys, Arg, etc.), an amino acid with a polar side chain substituted for another amino acid with a polar side chain (Asn, Cys, Gln, Ser, Thr. Tyr, etc.). In another embodiment, the variants can comprise the amino acid sequence of the native AOAH protein or polypeptide with at least one non-conservative amino acid substitution. Preferably, the non-conservative amino acid substitution enhances the enzymatic activity of the variant, such that the enzymatic activity of the variant is increased relative to the native AOAH protein or polypeptide.

In an embodiment, the MSC is transformed by a recombinant nucleic acid encoding the recombinant (i.e. exogenous) AOAH polypeptide. Thus, in another aspect, the present disclosure relates to a genetically-modified MSC comprising a recombinant (i.e. exogenous) nucleic acid encoding an AOAH polypeptide.

In an embodiment, the recombinant (i.e. exogenous) ANGPT1 polypeptide comprises or consists of a sequence that is at least 50, 60 or 70% identical to the amino acid sequence of mature native human ANGPT1 depicted in **FIG. 9A**/SEQ ID NO:7 (residues 16-498) and that exhibits the biological activity of native human ANGPT1, *e.g*., the ability to bind and activate the TEK/TIE2 receptor. In embodiments, the recombinant (i.e. exogenous) ANGPT1 polypeptide comprises or consists of a sequence that is at least 75%, 80%, 85%, 90%, 95%, 96%, 97%, 98% or 99% identical to the amino acid sequence of mature native human ANGPT1 depicted in **FIG. 9A**/SEQ ID NO:7 (residues 16-498) and that exhibits the biological activity of native human ANGPT1. In an embodiment, the recombinant (i.e. exogenous) ANGPT1 polypeptide comprises or consists of a sequence that is at least 70% identical to the amino acid sequence of full-length native human ANGPT1 depicted in **FIG. 9A**/SEQ ID NO:7 (residues 1-498) and that exhibits the biological activity of native human ANGPT1. In embodiments, the recombinant (i.e. exogenous) ANGPT1 polypeptide comprises or consists of a sequence that is at least 75%, 80%, 85%, 90%, 95%, 96%, 97%, 98% or 99% identical to the amino acid sequence of full-length native human ANGPT1 depicted in **FIG. 9A**/SEQ ID NO:7 (residues 1-498) and that exhibits the biological activity of native human ANGPT1.

Thus, the recombinant (i.e. exogenous) ANGPT1 polypeptide may be the native human ANGPT1 protein (full-length or mature form) or a biologically active variant thereof comprising amino acid deletions, substitutions and/or additions that do not significantly interfere with the biological activity. In an embodiment, the variant exhibits a biological activity that is at least 70%, 75%, 80%, 85%, 90%, 95% or 100% that of native human ANGPT1. In an embodiment, the variant does not comprise a mutation in a region involved in ANGPT1 oligomerization, for example a mutation in the super clustering domain (residues 81-119) or the central coiled domain (residues 153-261). In another embodiment, the variant does not comprise a mutation in the fibrinogen C-terminal domain (residues 277-497). In further embodiments, the variant does not comprise a mutation of position 119 (*e.g*., A119S) and/or position 249 (*e.g*., K249R). In another embodiment, the variant does not comprise a deletion of the five C-terminal amino acids.

In an embodiment, the variant comprises one or more conservative substitutions, as defined above for AOAH. Preferably, the non-conservative amino acid substitution enhances the biological activity of the variant, such that the biological activity of the variant is increased relative to the native ANGPT1 protein or polypeptide.

In an embodiment, the MSC is transformed by a recombinant nucleic acid encoding the recombinant (i.e. exogenous) ANGPT1 polypeptide. Thus, in another aspect, the present disclosure relates to a genetically-modified MSC comprising a recombinant (i.e. exogenous) nucleic acid encoding an ANGPT1 polypeptide.

As used herein, "nucleic acid" shall mean any nucleic acid molecule, including, without limitation, DNA, RNA and hybrids or modified variants thereof. An "exogenous" or "recombinant" nucleic acid relates to any nucleic acid introduced into the cell, which is not a component of the cells "original" or "natural" genome. Exogenous nucleic acids may be integrated or nonintegrated, or relate to stably transfected nucleic acids.

The recombinant nucleic acid may be a DNA or mRNA molecule comprising a nucleotide sequence encoding the recombinant (i.e. exogenous) AOAH polypeptide and/or the second polypeptide (*e.g*., ANGPT1). Methods for introducing recombinant nucleic acids into cell are well known in the art. Examples of such methods include, but are not limited to, the use of a lipid, protein, particle, or other molecule capable of facilitating cell transformation with the nucleic acid, electroporation. However, an MSC also can be contacted with a nucleic acid encoding AOAH and/or the second polypeptide (*e.g.*, ANGPT1) *in vivo*, such as by way of a gene gun, for example.

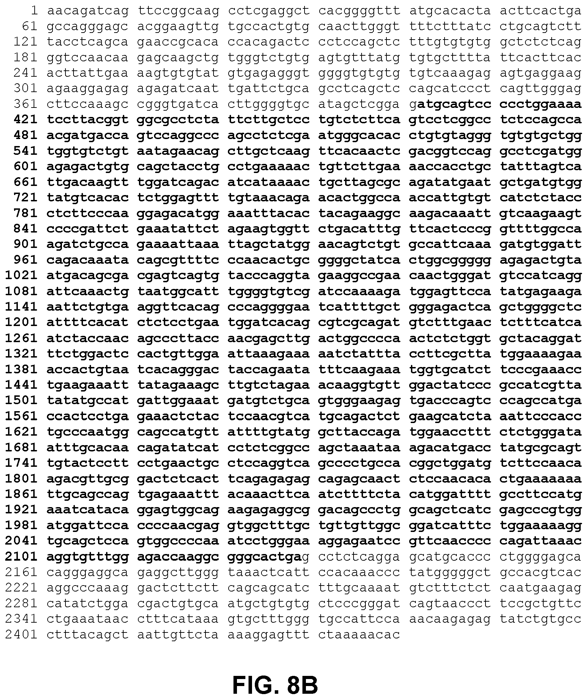

In an embodiment, the recombinant nucleic acid encoding AOAH comprises a nucleotide sequence that is at least 70% identical to the nucleotide sequence encoding the full-length, precursor or mature form of human AOAH depicted in **FIG. 8B**/SEQ ID NO:6. In embodiments, the recombinant nucleic acid encoding AOAH comprises a nucleotide sequence that is at least 75%, 80%, 85%, 90%, 95%, 96%, 97%, 98% or 99% identical to the nucleotide sequence encoding the full-length, precursor or mature form of human AOAH depicted in **FIG. 8B**/SEQ ID NO:6. In a further embodiment, the recombinant nucleic acid encoding AOAH comprises the nucleotide sequence encoding the full-length, precursor or mature form of human AOAH depicted in **FIG. 8B**/SEQ ID NO:6.

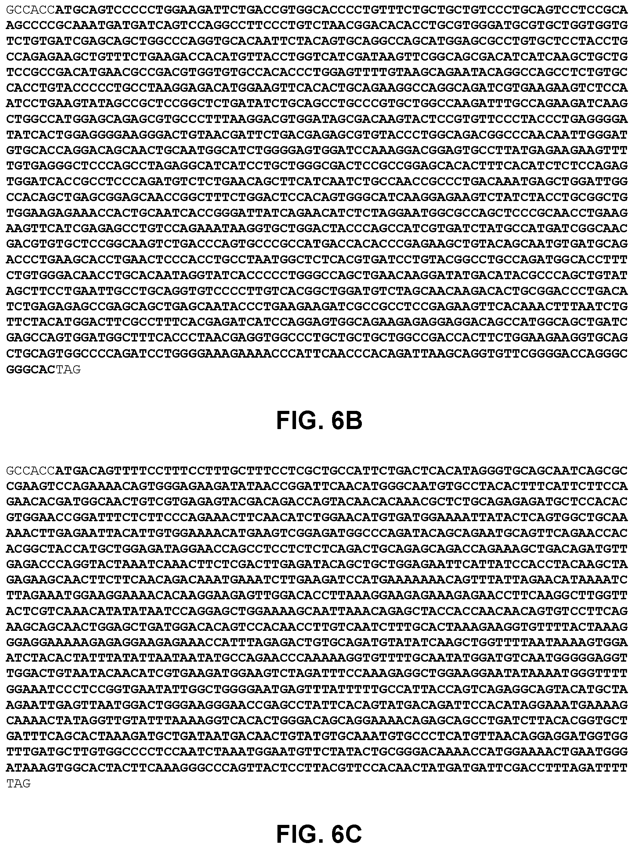

In an embodiment, the nucleic acid encoding AOAH comprises a codon-optimized nucleotide sequence encoding human AOAH. Without being bound to any particular theory or mechanism, it is believed that codon optimization of the nucleotide sequence increases the translation efficiency of the mRNA transcripts. Codon optimization of the nucleotide sequence may involve substituting a native codon for another codon that encodes the same amino acid, but can be translated by tRNA that is more readily available within a cell, thus increasing translation efficiency. Optimization of the nucleotide sequence may also reduce secondary mRNA structures that would interfere with translation, thus increasing translation efficiency. In this regard, the nucleic acid encoding AOAH may comprise the codon-optimized nucleotide sequence depicted in **FIG. 6B**/SEQ ID NO:1 (nucleotides 7-1734), or a sequence having at least 70%, 75%, 80%, 85%, 90%, 95%, 96%, 97%, 98% or 99% therewith.

In an embodiment, the recombinant nucleic acid encoding ANGPT1 comprises a nucleotide sequence that is at least 70% identical to the nucleotide sequence encoding the full-length, precursor or mature form of human ANGPT1 depicted in **FIGs. 9B-9C**/SEQ ID NO:8. In embodiments, the recombinant nucleic acid comprises a nucleotide sequence that is at least 75%, 80%, 85%, 90%, 95%, 96%, 97%, 98% or 99% identical to the nucleotide sequence encoding the full-length, precursor or mature form of human ANGPT1 depicted in **FIGs. 9B-****9C**/SEQ ID NO:8.

In an embodiment, the nucleic acid encoding ANGPT1 comprises a codon-optimized nucleotide sequence encoding human ANGPT1.

Methods, tools, platforms and services to synthesize codon-optimized nucleic acids are well known in the art, such as the OptimumGene^{™} platform from GenScript, the Codon Optimization Tool from Integrated DNA Technologies, the GeneOptimizer^{™} platform from ThermoFisher Scientific, OPTIMIZER from the NCBI (Nucleic Acids Res. 2007 Jul; 35(Web Server issue): W126-W131), Codon Optimization OnLine (COOL) (Bioinformatics, Volume 30, Issue 15, 1 August 2014, Pages 2210-2212), and the like.

In an embodiment, the recombinant nucleic acid is present in a vector or plasmid, i.e. a recombinant expression vector or plasmid. Thus, in another aspect, the present disclosure relates to a genetically modified MSC comprising a recombinant expression vector comprising the nucleic acid encoding an AOAH polypeptide as described herein. In an embodiment, the genetically modified MSC further comprises a recombinant expression vector comprising the nucleic acid encoding an ANGPT1 polypeptide as described herein. In an embodiment, the nucleic acids encoding AOAH and ANGPT1 are present in different recombinant expression vectors. In another embodiment, the nucleic acids encoding AOAH and ANGPT1 are present in the same recombinant expression vector.

The recombinant expression vector or plasmid can be any suitable recombinant expression vector or plasmid that contains a recombinant nucleic acid as described above, Suitable vectors include those designed for expression of a gene of interest in MCSs, which are well known in the art. In an embodiment, the recombinant expression vector or plasmid is a miniplasmid. Miniplasmids are small (∼4kb) circular plasmid derivatives that have been freed from all prokaryotic vector parts (i.e. they contain no bacterial DNA sequences) that used as transgene carriers for the genetic modification of mammalian cells. Miniplasmids and uses thereof are described, for example, in Minicircle and Miniplasmid DNA Vectors: The Future of Nonviral and Viral Gene Transfer, by Dr. Martin Schleef (editor), May 2013, Wiley-Blackwell, 258 pages.

In some embodiments, the recombinant expression vector is a viral vector. Genetically modified viruses have been widely applied for the delivery of genes into stem cells including MSCs. Preferred viral vectors for genetic modification of the MSCs described herein relate to retroviral vectors, in particular to gamma retroviral vectors. The gamma retrovirus (sometimes referred to as mammalian type C retroviruses) is a sister genus to the lentivirus clade, and is a member of the Orthoretrovirinae subfamily of the retrovirus family. The Murine leukemia virus (MLV or MuLV), the Feline leukemia virus (FeLV), the Xenotropic murine leukemia virus-related virus (XMRV) and the Gibbon ape leukemia virus (GALV) are members of the gamma retrovirus genus. A skilled person is aware of the techniques required for utilization of gamma retroviruses in genetic modification of MSCs. For example, the vectors described Maetzig et al. (Gamma retroviral vectors: biology, technology and application, 2001, Viruses 3(6):677-713) or similar vectors may be employed. For example, the Murine Leukemia Virus (MLV), a simple gammaretrovirus, can be converted into an efficient vehicle of genetic therapeutics in the context of creating gamma retrovirus-modified MSCs and expression of a therapeutic transgene from said MSCs after delivery to a subject. Genetically modified viruses have been widely applied for the delivery of genes into stem cells. Adenoviruses may be applied, or RNA viruses such as Lentiviruses, or other retroviruses. Adenoviruses have been used to generate a series of vectors for gene transfer cellular engineering. The initial generation of adenovirus vectors were produced by deleting the E1 gene (required for viral replication) generating a vector with a 4kb cloning capacity. An additional deletion of E3 (responsible for host immune response) allowed an 8kb cloning capacity. Further generations have been produced encompassing E2 and/or E4 deletions. Lentiviruses are members of Retroviridae family of viruses (M. Scherr et al., Curr Gene Ther. 2002 Feb; 2(1):45-55). Lentivirus vectors are generated by deletion of the entire viral sequence with the exception of the LTRs and cis acting packaging signals. The resultant vectors have a cloning capacity of about 8 kb. One distinguishing feature of these vectors from retroviral vectors is their ability to transduce dividing and non-dividing cells as well as terminally differentiated cells.

The recombinant expression vector or plasmid can comprise regulatory sequences, such as transcription and translation initiation and termination codons, which are specific to the type of host (e.g., an animal such as a human) into which the vector is to be introduced, as appropriate, and taking into consideration whether the vector is DNA- or RNA-based. The recombinant expression vector or plasmid can include one or more marker genes, which allow for selection of transformed or transfected hosts. Marker genes include biocide resistance, *e.g*., resistance to antibiotics, heavy metals, etc., complementation in an auxotrophic host to provide prototrophy, and the like, Suitable marker genes for the recombinant expression vectors include, for instance, neomycin G418 resistance genes, hygromycin resistance genes, tetracycline resistance genes, and ampicillin resistance genes, and the like. The recombinant expression vector or plasmid can comprise a native or normative promoter operably linked to the nucleic acid encoding AOAH and/or the second polypeptide (*e.g*., ANGPT1), and that is functional in MSCs. The selection of a promoter, *e.g*., strong, weak, inducible, tissue-specific and developmental-specific, is within the ordinary skill of the artisan. Similarly, the combining of a nucleotide sequence with a promoter is also within the skill of the artisan. The promoter cars be a non-viral promoter or a viral promoter, *e.g*., a cytomegalovirus (CMV) promoter, an SV40 promoter, an RSV promoter, or a promoter found in the long-terminal repeat of the murine stem cell virus. The promoter may comprise various motifs that enhance the binding of transcription factors such as initiator (INR), motif ten element (MTE) and downstream promoter element (DPE) motifs (see, *e.g*., Martinez, Transcription. 2012; 3(6): 295-299). The recombinant expression vector can be designed for either transient expression, for stable expression, or for both. Also, the recombinant expression vectors can be made for constitutive expression or for inducible expression. The vectors useful in the context of the disclosure can be "naked‴ nucleic acid vectors (i.e., vectors having little or no proteins, sugars, and/or lipids encapsulating them), or vectors complexed with other molecules. Other molecules that can be suitably combined with the vectors include without limitation viral coats, cationic lipids, liposomes, polyamines, gold particles, and targeting moieties such as ligands, receptors, or antibodies that target cellular molecules.

Any given gene delivery method is encompassed by the disclosure and preferably relates to viral or non-viral vectors as described above, as well as biological or chemical methods of transfection. The methods can yield either stable or transient gene expression in the system used. Non-viral methods include conventional plasmid transfer and the application of targeted gene integration through the use of genome editing technologies, as described above. These represent approaches for vector transformation that have the advantage of being both efficient, and often site-specific in their integration. Physical methods to introduce vectors into cells are known to a skilled person. One example relates to electroporation, which relies on the use of brief, high voltage electric pulses which create transient pores in the membrane by overcoming its capacitance. One advantage of this method is that it can be utilized for both stable and transient gene expression in most cell types. Alternative methods relate to the use of liposomes, protein transduction domains or other suitable reagents promoting nucleic acid entry in a cell. Appropriate methods are known to a skilled person and are not intended as limiting embodiments of the present disclosure.

Mesenchymal stem cells (MSCs, also referred to as mesenchymal stromal cells) are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum. MSC are capable of differentiating into different germ lines such as mesoderm, endoderm, and ectoderm. Thus, MSCs are capable of differentiating into a large number of cell types including, but not limited to, adipose, osseous, cartilaginous, elastic, muscular, and fibrous connective tissues. The specific lineagecommitment and differentiation pathway entered into by MSCs depends upon various influences, including mechanical influences and/or endogenous bioactive factors, such as growth factors, cytokines, and/or local microenvironmental conditions established by host tissues. MSCs are thus non-hematopoietic progenitor cells that divide to yield daughter cells that are either stem cells or are precursor cells that in time will irreversibly differentiate to yield a phenotypic cell. Examples of MSCs include mesenchymal precursor cells (MPCs).

MSCs are known to exhibit immune evasive properties after administration to a patient. MSCs have been shown to exhibit a beneficial immune modulatory effect in cases of transplantation of allogeneic donor material (Le Blanc et al., Lancet 2004: 363, p. 1439), thereby reducing a potentially pathogenic alloreactivity and rejection. The therapeutic delivery of MSCs can be performed via systemic injection, followed by MSC homing to and engraftment within sites of injury (Kidd et al., Stem Cells 2009: 27, p. 2614).

In some aspects, the MSCs can be progeny cells (which can also be referred to as expanded cells). Progeny cells can be produced from the *in vitro* culture of stem cells. Expanded cells of the disclosure may have a wide variety of phenotypes depending on the culture conditions (including the number and/or type of stimulatory factors in the culture medium), the number of passages and the like. In certain embodiments, the progeny cells are obtained after about 2, about 3, about 4, about 5, about 6, about 7, about 8, about 9, or about 10 passages from the parental population. However, the progeny cells may be obtained after any number of passages from the parental population. And the progeny cells can be obtained by culturing in a suitable culture medium.

In one embodiment, the progeny cells are multipotential expanded MSC progeny (MEMPs) as described in WO 2006/032092. Methods for preparing enriched populations of MSC from which progeny may be derived are described in WO 01/04268 and WO 2004/085630. In an *in vitro* context, MSCs will rarely be present as an absolutely pure preparation and will generally be present with other cells that are tissue-specific committed cells (TSCCs). WO 01/04268 refers to harvesting such cells from bone marrow at purity levels of about 0.1% to 90%. The population comprising MSC from which progeny are derived may be directly harvested from a tissue source, or alternatively it may be a population that has already been expanded *ex vivo.*

For example, the progeny may be obtained from a harvested, unexpanded, population of substantially purified MSC, comprising at least about 0.1, 1, 5, 10, 20, 30, 40, 50, 60, 70, 80 or 95% of total cells of the population in which they are present. This level may be achieved, for example, by selecting for cells that are positive for at least one marker typically expressed on MSCs such as CD29, CD44, CD90, CD49a-f, CD51, CD73 (SH3), CD105 (SH2), CD106, CD166, and Stro-1, and/or negative for markers such as CD45, CD34, CD14 or CD11b, CD79a or CD19 and HLA-DR.

For example, the progeny may be obtained from a harvested, unexpanded, population of substantially purified MSC, comprising at least about 0.1, 1, 5, 10, 20, 30, 40, 50, 60, 70, 80 or 95% of total cells of the population in which they are present. This level may be achieved, for example, by selecting for cells that are positive for at least one marker selected from CD29, CD44, CD90, CD49a-f, CD51, CD73 (SH3), CD105 (SH2), CD106, CD166, and Stro-1.

The MSC starting population may be derived from any one or more tissue types set out in WO 01/04268 or WO 2004/085630, namely bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon, and skeletal muscle.

MEMPS can be distinguished from freshly harvested MSCs in that they are positive for the marker Stro-1 and negative for the marker Alkaline phosphatase (ALP). In contrast, freshly isolated MSCs are positive for both Stro-1 and ALP. In a preferred embodiment of the present disclosure, at least 15%, 20%, 30%, 40%, 50%, 60%, 70%, 80%, 90% or 95% of the administered cells have the phenotype Stro-1⁺, ALP-.

Reference to a cell "positive" (also "+") for a given marker means that it may be either a low (lo or dim) or a high (bright, bri) expresser of that marker depending on the degree to which the marker is present on the cell surface, where the terms relate to intensity of fluorescence or other color used in the color sorting process of the cells. The distinction of lo (or dim or dull) and bri will be understood in the context of the marker used on a particular cell population being sorted. Reference to a cell as being "negative" (or "-") for a given marker, does not mean that the marker is not expressed at all by that cell. It means that the marker is expressed at a relatively very low level by that cell, and that it generates a very low signal when detectably labeled.

In one embodiment, the cells are taken from a patient with SIRS or sepsis, or a subject not suffering from SIRS or sepsis, genetically-modified to overexpress AOAH (and optionally a second polypeptide, such as ANGPT1), and optionally cultured *in vitro* using standard techniques, and administered to a patient in need of treatment as an autologous or allogeneic transplant. In an alternative embodiment, cells of one or more of the established human cell lines are used. In another useful embodiment of the disclosure, cells of a non-human animal (or if the patient is not a human, from another species) are used.

The present technology can be practiced using cells from any non-human animal species, including but not limited to non-human primate cells, ungulate, canine, feline, lagomorph, rodent, avian, and fish cells. Primate cells with which the disclosure may be performed include but are not limited to cells of chimpanzees, baboons, cynomolgus monkeys, and any other New or Old World monkeys. Ungulate cells with which the disclosure may be performed include but are not limited to cells of bovines, porcines, ovines, caprines, equines, buffalo and bison. Rodent cells with which the disclosure may be performed include but are not limited to mouse, rat, guinea pig, hamster and gerbil cells. Examples of lagomorph species with which the disclosure may be performed include domesticated rabbits, jack rabbits, hares, cottontails, snowshoe rabbits, and pikas. Chickens (*Gallus gattus*) are an example of an avian species with which the disclosure may be performed.

MSCs can be stored before use. Methods and protocols for preserving and storing of eukaryotic cells, and in particular mammalian cells, are well known in the art (see, for example, Pollard, J. W. and Walker, J. M. (1997) Basic Cell Culture Protocols, Second Edition, Humana Press, Totowa, N.J.; Freshney, R. I. (2000) Culture of Animal Cells, Fourth Edition, Wiley-Liss, Hoboken, N.J.). Any method maintaining the biological activity of the isolated stem cells such as mesenchymal stem/progenitor cells, or progeny thereof, may be utilized in connection with the present disclosure. In one preferred embodiment, the cells are maintained and stored by using cryo-preservation.

MSCs can be obtained using a variety of techniques. For example, a number of cellsorting techniques by which cells are physically separated by reference to a property associated with the cell-antibody complex, or a label attached to the antibody can be used. This label may be a magnetic particle or a fluorescent molecule. The antibodies may be cross-linked such that they form aggregates of multiple cells, which are separable by their density. Alternatively, the antibodies may be attached to a stationary matrix, to which the desired cells adhere.

A "culture medium" as used herein, encompasses (a) both a culture medium that contains the typical components used for culturing a MSC, such as amino acids, glucose, and various salts, with or without the MSC, and (b) a composition isolated from the culture medium, including a composition comprising components released from the MSC during the culturing. The culture medium may contain components that are solid, liquid, gaseous or a mixture of phases and materials. Culture medium components include, but are not limited to, agar, agarose, gelatin and collagen matrices. "Culture medium" includes material that is intended for use in a cell culture, even if it has not yet been contacted with cells. For example, a nutrient rich liquid prepared for bacterial culture can be a culture medium.

In an embodiment, the MSCs are contacted with or exposed to one or more agents during or after culture/expansion (and prior to administration/use in the subject) to confer them with a desired property, *e.g.* to induce the expression/secretion of anti-inflammatory mediators and/or to inhibit the expression/secretion of pro-inflammatory mediators. For example, MSCs exposed to low concentration prostacyclins (*e.g*., treprostinil), for example about 10 µg/mL of treprostinil, have been shown to exhibit enhanced secretion of certain anti-inflammatory mediators such as IL-10, IL-13, IDO, iNOS, HLA and TGFβ, and reduced secretion of pro-inflammatory mediators such as TNFα and IL-4, by MSCs (see, *e.g*., WO 2018/080990). Such exposure to the one or more agents may be for any suitable time to induce the desired effect, for example at least 6, 12, 18, 24, 36 or 48 hours.

In another aspect, the present disclosure relates to the genetically-modified MSC described herein, or a pharmaceutical composition comprising same, for use as a medicament. In an embodiment, the medicament is for the prevention or treatment of SIRS, for example SIRS caused by sepsis, in a subject. The present disclosure also relates to a method for preventing or treating systemic inflammatory response syndrome (SIRS) comprising administering to a subject in need thereof an effective amount of the genetically-modified MSC described herein, or a pharmaceutical composition comprising same. The present disclosure also relates to the use of the genetically-modified MSC described herein, or a pharmaceutical composition comprising same, for preventing or treating SIRS in a subject. The present disclosure also relates to the use of the genetically-modified MSC described herein, or a pharmaceutical composition comprising same, for the manufacture of a medicament for preventing or treating SIRS in a subject.

As used herein the terms "treating", "treat" or "treatment" include eliminating, ameliorating, alleviating, or abating a disease or condition or one or more symptoms thereof, whether or not the disease or condition is considered to be "cured" or "healed" and whether or not all symptoms are resolved. The terms also include reducing or preventing progression of a disease or condition or one or more symptoms thereof, impeding or preventing an underlying mechanism of a disease or condition or one or more symptoms thereof, and achieving any therapeutic and/or prophylactic benefit.

As used herein the terms "preventing", "prevent" or "prevention" include reducing the occurrence and/or severity of a disease or condition or one or more symptoms thereof in a subject (e.g., a subject at risk of suffering from SIRS/sepsis) relative to an untreated control subject, or delaying the onset of one or more symptoms of the disease or condition in a subject (e.g., a subject at risk of suffering from SIRS/sepsis) relative to the untreated control subject.

The term "systemic inflammatory response syndrome" or "SIRS" refers to a condition related to systemic inflammation in response to an insult such as an infection, a trauma, burns, pancreatitis, or a variety of other injuries, and that results in multiple organ dysfunctions; acute lung injury (ALI) represents the most common and earliest organ failure. SIRS may occur as a consequence of various pathogenic events, with sepsis syndrome being the most prevalent and lethal cause. SIRS is identified by two or more symptoms including fever or hypothermia, tachycardia, tachypnoea and change in blood leucocyte count.

In an embodiment, the SIRS is associated or caused by sepsis. The term "sepsis" is applied to a number of diseases, conditions and/or syndromes which may have an infectious (for example viral, bacterial and/or fungal) etiology. It encompasses sepsis (which is often defined as "SIRS in response to an infectious process"), severe sepsis (that is sepsis with sepsis-induced organ dysfunction or tissue hypoperfusion (which itself might manifest as hypotension, elevated lactate or decreased urine output) and septic shock (severe sepsis plus persistently low blood pressure despite, for example, the administration of intravenous fluids).

The term "sepsis" is most often applied to diseases, conditions and/or syndromes which result from "bacterial sepsis". Bacterial sepsis may stem from the presence of bacteria in blood and may sometimes be referred to as "bacteremia" or "septicemia". The term "sepsis" may also embrace diseases and/or conditions which are caused or contributed to by the presence of bacterial components such as LPS, toxins and/or membrane fragments in the blood. Components of this type may originate from primary infections present in other tissues and/or organs, for example, infections present in the lungs, brain, skin, urinary tract, pelvis and/or abdomen. Sepsis can be a very severe condition which occasionally leads to multiple organ failure and death. There may be a number of pathologies and/or symptoms associated with each type of sepsis and these will be collectively referred to hereinafter as "sepsis associated pathologies". For example, sepsis associated pathologies may include, for example, fever, increased heart rate, increased rate of respiration and/or low blood pressure. The primary mechanism which underpins the pathology of sepsis and many of the symptoms and outcomes associated therewith is an exacerbated, exaggerated and/or inappropriate host immune response. This exacerbated immune response (which is the body's response to infection) leads to host tissue damage and/or organ damage/failure. The exacerbated or inappropriate immune response may include the dysregulation of innate immune responses as well as the production and/or over production of certain pro-coagulation cytokines (including, for example, tumor necrosis factor, interleukin-1 (IL-1) and interleukin-6 (IL-6)).

In an embodiment, the treatment reduces one or more symptoms of SIRS or sepsis. In an embodiment, the treatment reduces the levels (*e.g*., plasmatic levels) of one or more pro-inflammatory cytokines such as IL-6, IL-10, IL-12, MIP-1α (CCL3), eotaxin, G-CSF, KC and/or MCP-1, in the subject. In a further embodiment, the treatment reduces the levels (*e.g*., plasmatic levels) of at least one of IL-10, MIP-1α, and MCP-1, and preferably the treatment reduces the levels (*e.g*., plasmatic levels) of IL-10, MIP-1α, and MCP-1.

As used herein, the term "subject" (also referred to herein as a "patient") includes warm-blooded animals, preferably mammals, including humans. In a preferred embodiment, the subject is a primate. In an even more preferred embodiment, the subject is a human. In an embodiment, the subject has one or more symptoms of SIRS or sepsis. In another embodiment, the subject is at risk of developing SIRS or sepsis.

In an embodiment, the genetically-engineered MSCs described herein are present in a composition, preferably a pharmaceutical composition. Thus, in another aspect, the present disclosure provides a pharmaceutical composition comprising the genetically-engineered MSCs described herein. In an embodiment, the pharmaceutical composition comprises a therapeutically effective amount of the genetically-engineered MSCs described herein.

In some embodiments, the pharmaceutical composition further comprises at least one pharmaceutically-acceptable carrier or excipient. The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically-acceptable carrier or excipient " as used herein means a pharmaceutically-acceptable material, composition or vehicle, such as a liquid or solid filler, diluent, excipient, or solvent encapsulating material.

Pharmaceutically acceptable carriers or excipients include saline, aqueous buffer solutions, solvents and/or dispersion media. The use of such carriers or excipients are well known in the art. The solution is preferably sterile and fluid to the extent that easy syringability exists. Preferably, the solution is stable under the conditions of manufacture and storage and preserved against the contaminating action of microorganisms such as bacteria and fungi through the use of preservatives, for example, parabens, chlorobutanol, phenol, ascorbic acid, thimerosal, and the like.

Some examples of materials and solutions which can serve as pharmaceutically-acceptable carriers or excipients include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; lubricants such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH-buffered solutions; polyesters, polycarbonates and/or polyanhydrides; antioxidants; antimicrobials; and other non-toxic compatible substances employed in pharmaceutical formulations.

The pharmaceutical compositions useful for the methods of the disclosure may comprise a polymeric carrier or extracellular matrix. A variety of biological or synthetic solid matrix materials (*e.g*., solid support matrices, biological adhesives or dressings, and biological/medical scaffolds) are suitable for use in this disclosure. The matrix material is preferably medically acceptable for use in *in vivo* applications. Non-limiting examples of such medically acceptable and/or biologically or physiologically acceptable or compatible materials include, but are not limited to, solid matrix materials that are absorbable and/or non-absorbable, such as small intestine submucosa (SIS), *e.g*., porcine-derived (and other SIS sources); crosslinked or non-crosslinked alginate, hydrocolloid, foams, collagen gel, collagen sponge, polyglycolic acid (PGA) mesh, polyglactin (PGL) mesh, fleeces, foam dressing, bioadhesives (*e.g*., fibrin glue and fibrin gel) and dead de-epidermized skin equivalents in one or more layers.

Suitable polymeric carriers include porous meshes or sponges formed of synthetic or natural polymers, as well as polymer solutions. One form of matrix is a polymeric mesh or sponge; the other is a polymeric hydrogel. Natural polymers that can be used include proteins such as collagen, albumin, and fibrin; and polysaccharides such as alginate and polymers of hyaluronic acid. Synthetic polymers include both biodegradable and non-biodegradable polymers. Examples of biodegradable polymers include polymers of hydroxy acids such as polylactic acid (PLA), polyglycolic acid (PGA), and polylactic acid-glycolic acid (PLGA), polyorthoesters, polyanhydrides, polyphosphazenes, and combinations thereof. Nonbiodegradable polymers include polyacrylates, polymethacrylates, ethylene vinyl acetate, and polyvinyl alcohols.

Polymers that can form ionic or covalently crosslinked hydrogels which are malleable are used to encapsulate cells. A hydrogel is a substance formed when an organic polymer (natural or synthetic) is cross-linked via covalent, ionic, or hydrogen bonds to create a three-dimensional open-lattice structure which entraps water molecules to form a gel.

Examples of materials which can be used to form a hydrogel include polysaccharides such as alginate, polyphosphazines, and polyacrylates, which are crosslinked ionically, or block copolymers such as Pluronics^{™} or Tetronics^{™}, polyethylene oxide-polypropylene glycol block copolymers which are crosslinked by temperature or pH, respectively. Other materials include proteins such as fibrin, polymers such as polyvinylpyrrolidone, hyaluronic acid and collagen.

In general, these polymers are at least partially soluble in aqueous solutions, such as water, buffered salt solutions, or aqueous alcohol solutions, that have charged side groups, or a monovalent ionic salt thereof. Examples of polymers with acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups. Examples of polymers with basic side groups that can be reacted with anions are poly(vinyl amines), poly(vinyl pyridine), poly(vinyl imidazole), and some imino substituted polyphosphazenes. The ammonium or quaternary salt of the polymers can also be formed from the backbone nitrogens or pendant imino groups. Examples of basic side groups are amino and imino groups.

In some embodiments, the pharmaceutical composition can comprise at least one additional therapeutic agent. For example, the composition may contain an analgesic to aid in treating inflammation or pain, or an anti-infective agent to prevent infection of the site treated with the composition. More specifically, non-limiting examples of useful therapeutic agents include the following therapeutic categories: analgesics, such as nonsteroidal antiinflammatory drugs, opiate agonists and salicylates; anti-infective agents, such as antihelmintics, antianaerobics, antibiotics, aminoglycoside antibiotics, antifungal antibiotics, cephalosporin antibiotics, macrolide antibiotics, miscellaneous β-lactam antibiotics, penicillin antibiotics, quinolone antibiotics, sulfonamide antibiotics, tetracycline antibiotics, antimycobacterials, antituberculosis antimycobacterials, antiprotozoals, antimalarial antiprotozoals, antiviral agents, anti-retroviral agents, scabicides, anti-inflammatory agents, corticosteroid anti-inflammatory agents, antipruritics/local anesthetics, topical anti -infectives, antifungal topical anti-infectives, antiviral topical anti-infectives; electrolytic and renal agents, such as acidifying agents, alkalinizing agents, diuretics, carbonic anhydrase inhibitor diuretics, loop diuretics, osmotic diuretics, potassium-sparing diuretics, thiazide diuretics, electrolyte replacements, and uricosuric agents; enzymes, such as pancreatic enzymes and thrombolytic enzymes; gastrointestinal agents, such as antidiarrheals, gastrointestinal anti-inflammatory agents, gastrointestinal anti -inflammatory agents, antacid anti-ulcer agents, gastric acid-pump inhibitor anti-ulcer agents, gastric mucosal anti-ulcer agents, H2-blocker anti-ulcer agents, cholelitholytic agent's, digestants, emetics, laxatives and stool softeners, and prokinetic agents; general anesthetics, such as inhalation anesthetics, halogenated inhalation anesthetics, intravenous anesthetics, barbiturate intravenous anesthetics, benzodiazepine intravenous anesthetics, and opiate agonist intravenous anesthetics; hormones and hormone modifiers, such as abortifacients, adrenal agents, corticosteroid adrenal agents, androgens, anti-androgens, immunobiologic agents, such as immunoglobulins, immunosuppressives, toxoids, and vaccines; local anesthetics, such as amide local anesthetics and ester local anesthetics; anti-inflammatory agents including corticosteroid anti-inflammatory agents, gold compound anti-inflammatory agents, immunosuppressive anti-inflammatory agents, nonsteroidal anti-inflammatory drugs (NSAIDs), salicylate anti-inflammatory agents, minerals; and vitamins, such as vitamin A, vitamin B, vitamin C, vitamin D, vitamin E, and vitamin K.

According to one embodiment of the present disclosure, the compositions can be co-administered with at least one other medicine for SIRS or sepsis. For example, the pharmaceutical compositions can be co-administered with antibiotics, intravenous fluid, corticosteroids, insulin, pain killers, sedatives, and/or vasopressors, for example. The combination of prophylactic/therapeutic agents and/or compositions of the present disclosure may be administered or co-administered (*e.g*., consecutively, simultaneously, at different times) in any conventional dosage form. Co-administration in the context of the present disclosure refers to the administration of more than one therapeutic in the course of a coordinated treatment to achieve an improved clinical outcome. Such co-administration may also be coextensive, that is, occurring during overlapping periods of time. For example, a first agent may be administered to a patient before, concomitantly, before and after, or after a second active agent is administered. The agents may in an embodiment be combined/formulated in a single composition and thus administered at the same time.

Compositions useful for the methods of the present disclosure may include cell culture components, *e.g*., culture media including amino acids, metals, coenzyme factors, as well as small populations of other cells, *e.g*., some of which may arise by subsequent differentiation of the stem cells.

Compositions useful for the methods of the present disclosure may be prepared, for example, by sedimenting out the subject cells from the culture medium and re-suspending them in the desired solution or material. The cells may be sedimented and/or changed out of the culture medium, for example, by centrifugation, filtration, ultrafiltration, etc.

The skilled artisan can readily determine the amount of MSCs and optional carrier(s) in compositions and to be administered in methods of the disclosure. Of course, for any composition to be administered to an animal or human, and for any particular method of administration, it is preferred to determine therefore: toxicity, such as by determining the lethal dose (LD) and LD₅₀ in a suitable animal model *e.g*., rodent such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

In an embodiment, the following amounts and ranges of amounts of MSCs are administered to the subject: (i) from about 1 × 10² to about 1 × 10⁸ cells/kg body weight; (ii) from about 1 × 10³ to about 1 × 10⁷ cells/kg body weight; (iii) from about 1 × 10⁴ to about 1 × 10⁶ cells/kg body weight; (iv) from about 1 × 10⁴ to about 1 × 10⁵ cells/kg body weight; (v) from about 1 × 10⁵ to about 1 × 10⁶ cells/kg body weight; (vi) from about 5 × 10⁴ to about 0.5 × 10⁵cells/kg body weight; (vii) about 1 × 10³ cells/kg body weight; (viii) about 1 × 10⁴ cells/kg body weight; (ix) about 5 × 10⁴ cells/kg body weight; (x) about 1 × 10⁵cells/kg body weight; (xi) about 5 × 10⁵ cells/kg body weight; (xii) about 1 × 10⁶ cells/kg body weight; and (xiii) about 1 × 10⁷ cells/kg body weight. Human body weights envisioned include, without limitation, about 5 kg, 10 kg, 15 kg, 30 kg, 50 kg, about 60 kg; about 70 kg; about 80 kg, about 90 kg; about 100 kg, about 120 kg and about 150 kg. The MSCs may be administered either in a bolus form, i.e. injection of all the cells during a short period of time, or it may be accomplished by a continuous administration (e.g., infusion) of small numbers of cells over a long period of time, or alternatively by administration of limited size boluses on several occasions over a period of time.

Compositions useful for the methods of the present disclosure can be administered via, *inter alia*, localized injection, including catheter administration, systemic injection, localized injection, intravenous injection, intrauterine injection or parenteral administration. When administering a therapeutic composition described herein (*e.g*., a pharmaceutical composition), it will generally be formulated in a unit dosage injectable form (solution, suspension, emulsion).

### MODE(S) FOR CARRYING OUT EMBODIMENTS OF THE DISCLOSURE

The present disclosure is illustrated in further details by the following non-limiting examples.

### Example 1: Construction of plasmids for expression of AOAH in MSC

**Materials:** AOAH gene was synthesized by GenScript, Restriction endonuclease Nhel, Sall, BamHI, and EcoRI were purchased from New England Biolabs, T4 DNA Ligase was purchased from New England Biolabs.

**Methods:** After AOAH gene was synthesized, gene sequence was checked by gene sequencing. Then the target gene was cut by restriction endonucleases at 37°C for 2h, and inserted into parental cloning vector by using T4 DNA Ligase and incubating at 16°C overnight. The parental AOAH plasmid was transformed into competent *E*. *coli* for amplification and miniplasmid preparation.

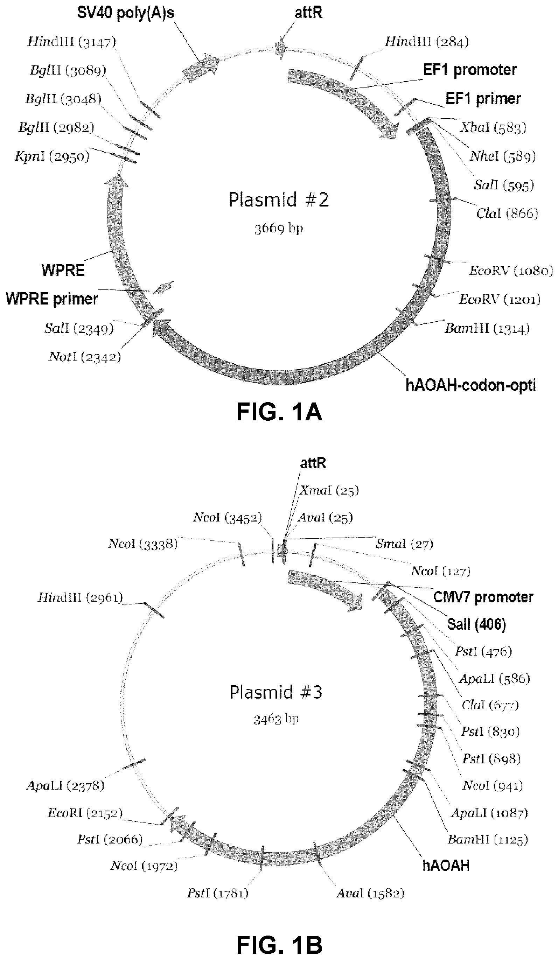

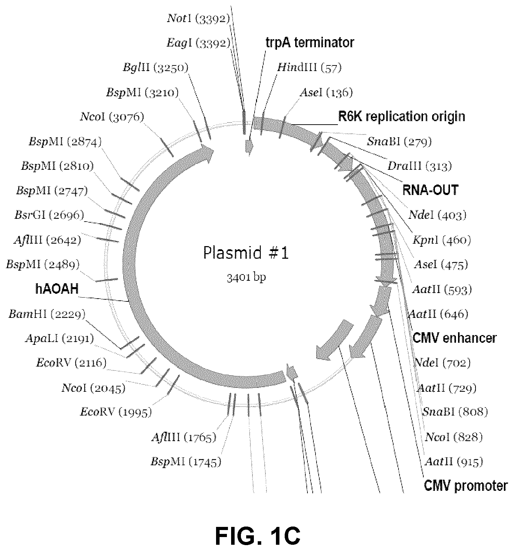

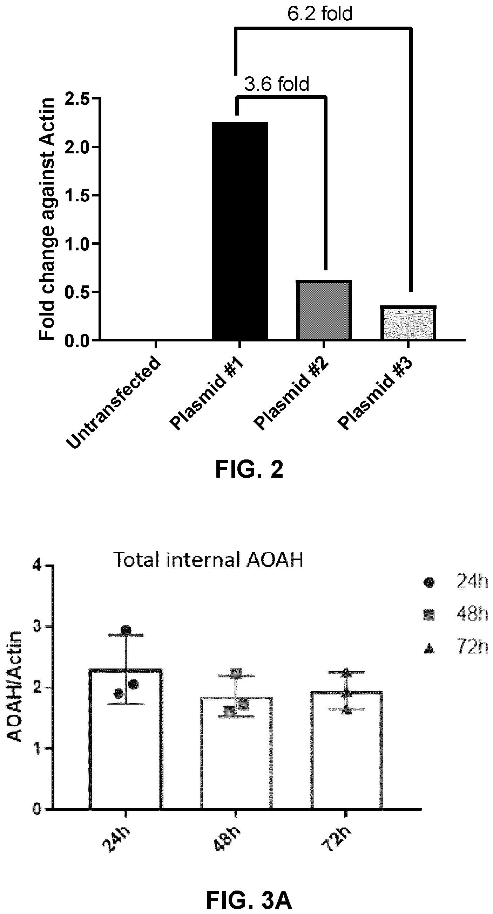

Three different plasmids were constructed and tested for expression of AOAH in MSC, namely a plasmid comprising the AOAH coding sequence under the control of an EF-1 promoter (plasmid #2, **FIG. 1A**), a plasmid comprising the AOAH coding sequence under the control of an CMV promoter (plasmid #3, **FIG. 1B**), and another plasmid comprising the AOAH coding sequence under the control of a CMV promoter/enhancer (plasmid #1, **FIG. 1C**). MCS were transfected with the different plasmids and the intracellular expression level of AOAH were measured on cell lysates by Western Blot. The results depicted in **FIG. 2** show that the MCS transfected with plasmid #1 express AOAH at levels that are 3.6- and 6.2-fold higher than MCS transfected with plasmid #2 and plasmid #3, respectively. Plasmid #1 was thus selected to perform the next experiments.

### Example 2: Expression and secretion of AOAH in MSC

**Material:** Anti-AOAH antibody was purchased from ThermoFisher, anti-AOAH ELISA kit was purchased from MyBioSource.

**Method:** MSC were seeded at the density of 10,000/cm² into T75 flask. 16-20 h postseeding, cells were transfected with plasmid #1 (~1µg/100,000 cells). After transfection, cells were cultured for 24h. 40 µg of cell lysates was used for Western Blot. Culture medium was harvested and assayed by ELISA.

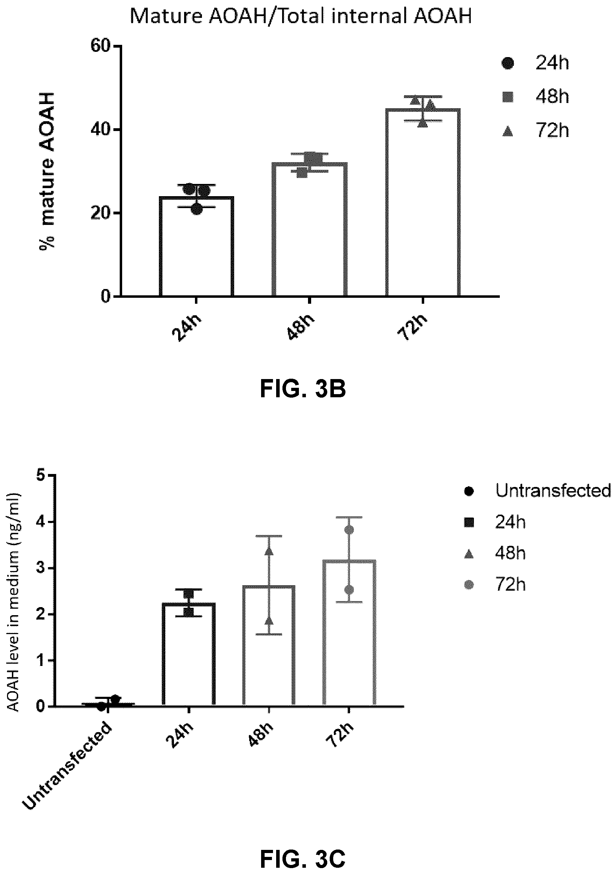

In order to determine the duration of the overexpression of AOAH in MSC, plasmid #1 was transfected into MSC, and Western Blots were performed on the cell lysates harvested at 24h, 48h, and 72h post-transfection. It was found that the total level of intracellular AOAH (precursor and mature form) was slightly lower at 72h relative to 24h post-transfection (**FIG. 3A**). However, the proportion of the mature form of AOAH (relative to total AOAH) was higher at 72h relative to 24h post-transfection (~45% vs ~24%, **FIG. 3B**). Thus, the transfection of MSC with plasmid #1 leads to a sustained expression of mature AOAH in these cells. The results presented in **FIG. 3C** show a gradual increase from 24h to 72h in the levels of AOAH in the culture medium of MSC transfected with plasmid #1.

### Example 3: Effect of administration of MSC transfected with the plasmid #1 encoding AOAH in murine models of sepsis

**Material:** 10-week-old C57BL/6 female mice were purchased from Charles River. LPS was purchased from Sigma, Plasma-Lyte A was purchased from Baxter, human albumin was purchased from Sigma, mouse cytokine Bio-Plex kit was purchased from Bio-Rad. Bio-Plex^{™} 200 System and Bio-Plex Pro^{™}∥ Wash Station were purchased from Bio-Rad.

**Methods:** Sepsis was induced with either endotoxin (LPS) or pathogenic *E*. *coli.* LPS was diluted with sterilized saline solution, and injected intraperitoneally into mice at a dose of 15 mg/kg. Cryopreserved pathogenic *E*. *coli* was diluted with sterilized saline solution, and injected intraperitoneally into mice at a dose of 1.2×10⁶ CFU per mouse. 1h post- IP injection, cells were delivered by intrajugular injection at a dose of 250,000 cells per mouse. In endotoxin-induced sepsis study, 20h post-LPS injection, 500 µl of blood from each mouse was drawn by a syringe which was prefilled with 50 µl of 50 mM EDTA. The needle was removed from the syringe, and the blood was gently expelled into a microfuge tube. The blood was centrifuged at 1700 × g, 10 min at 4°C. The upper plasma layer was transferred to a new microfuge tube, and centrifuged at 11,000xg, 5 min at 4°C to remove any residual contaminating debris/platelets. The plasma was transferred to a new microfuge tube after centrifuge, and frozen at -80°C to preserve or determine cytokine levels. Cytokine levels were determined by Bio-Plex according to the manufacturer's protocols.

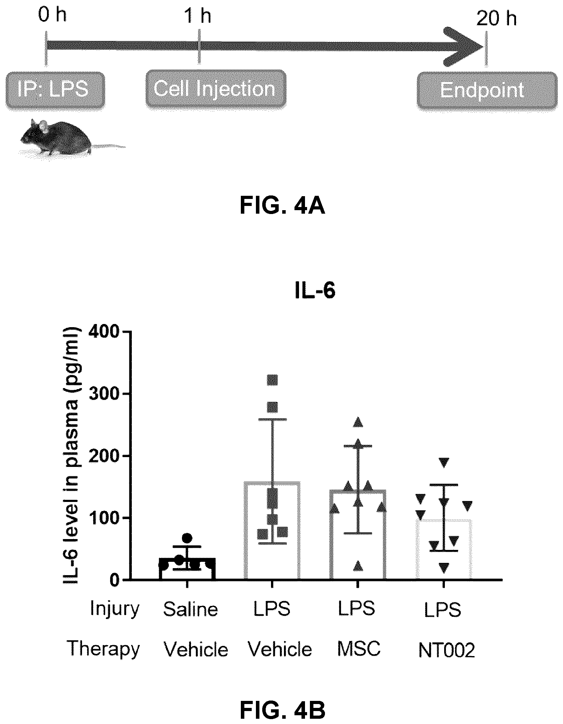

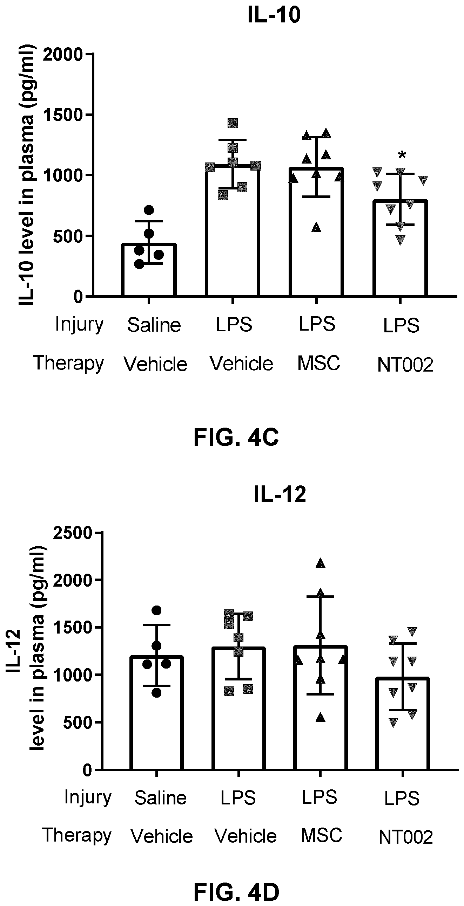

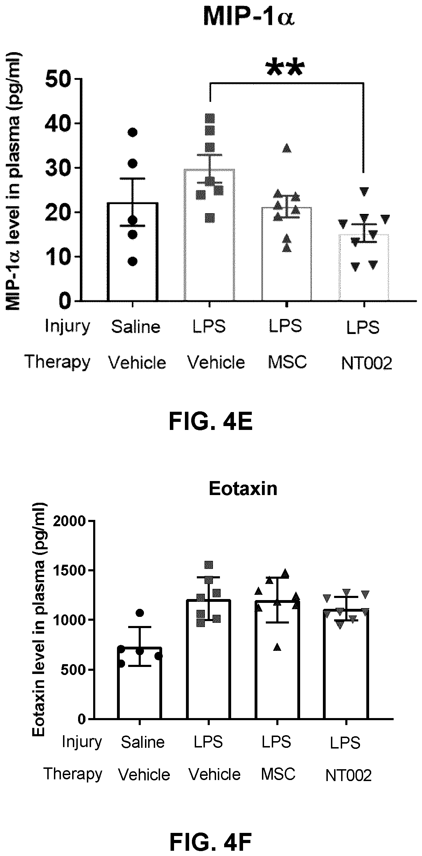

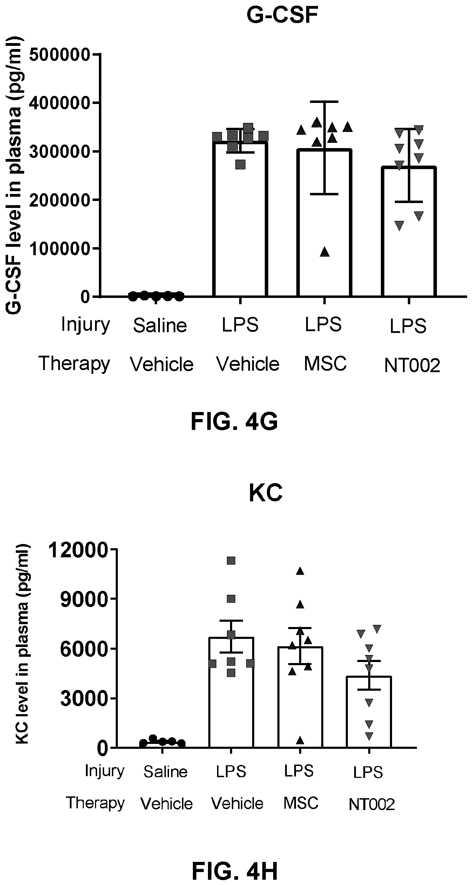

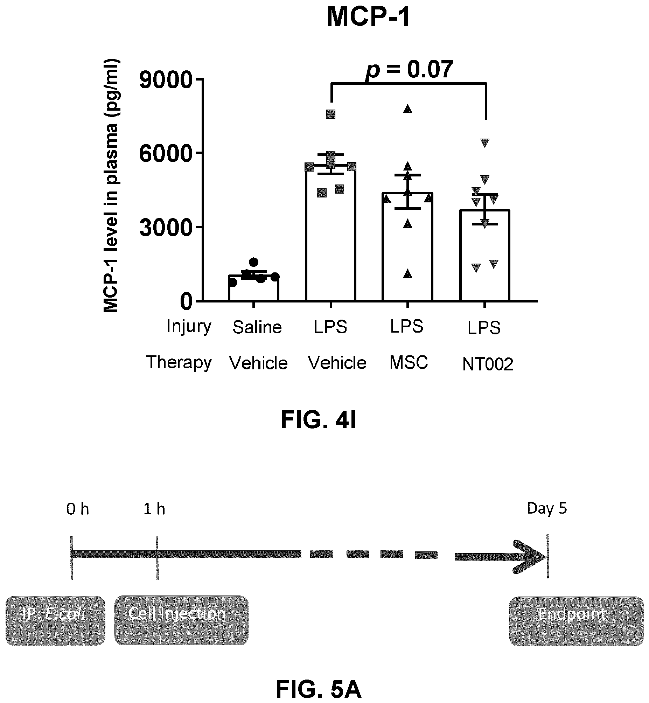

It was tested whether MSC transfected with plasmid #1 could reduce inflammation in LPS-induced endotoxemia, a murine model of sepsis. Endotoxemia was induced by intraperitoneal injection of LPS, and the mice were treated 1h post-LPS injection with NT002, untransfected MSC or saline vehicle. The plasmatic levels of several pro-inflammatory cytokines and chemokines was assessed 20h post-LPS injection. The results shown in **FIGs. 4B** to **4I** demonstrate that the administration of NT002 attenuates the increases in the plasmatic levels of certain pro-inflammatory cytokines and chemokines induced by LPS injection, thus providing evidence that MSC overexpressing AOAH may be useful for treating or reducing the severity of the systemic inflammatory response syndrome (SIRS) induced by sepsis.

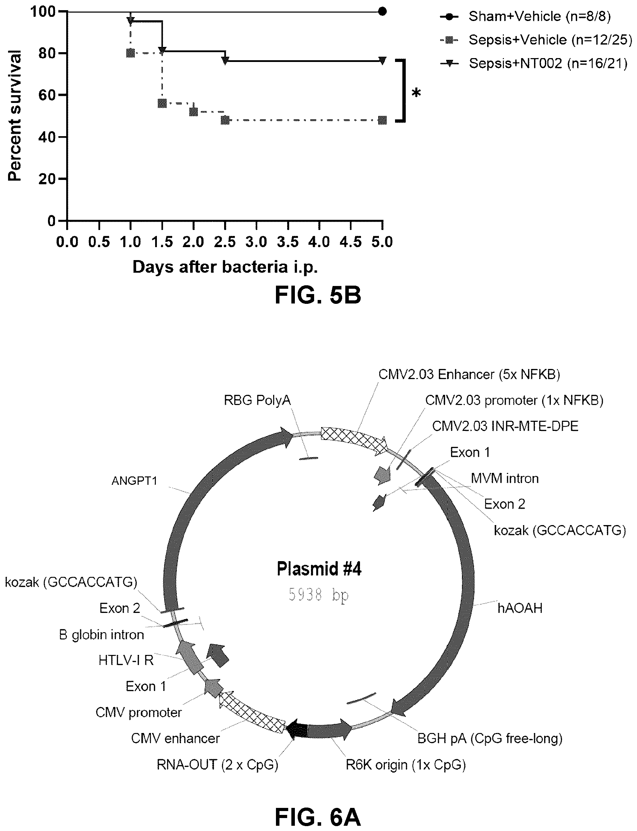

In pathogenic *E. coli*-induced sepsis study, animals were observed for 5 days post-bacteria inoculation to determine the survival rate (**FIG. 5A**). The results shown in **FIG. 5B** demonstrate that the administration of NT002 protects the mice from a high mortality (~50%) induced by the pathogenic *E*. *coli* inoculation.

### Example 4: Effect of administration of MSC overexpressing AOAH and ANGPT1 in murine models of sepsis

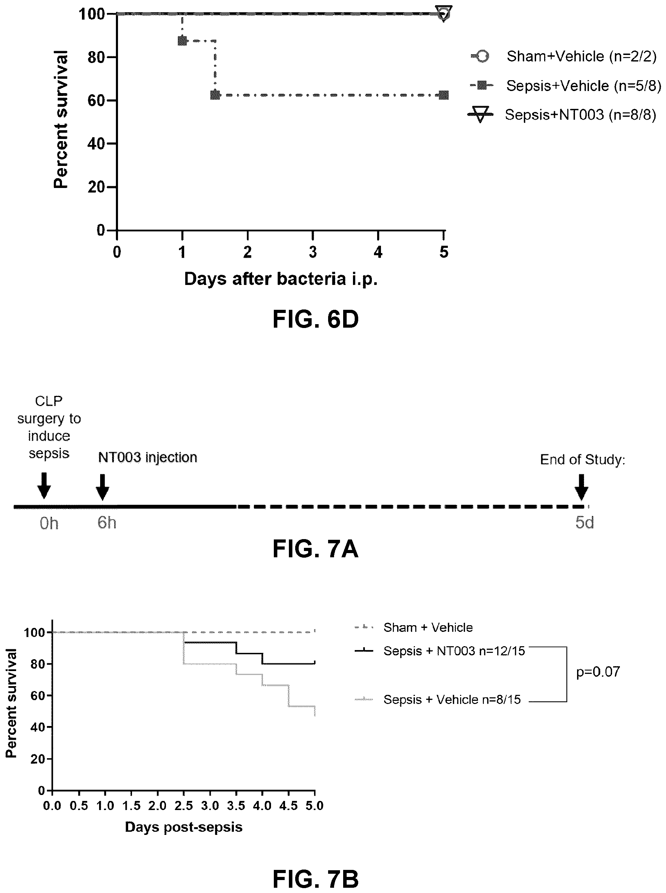

MSCs were transfected with plasmid #4 (**FIG. 6A**) that comprises a codon-optimized sequence encoding hAOAH (**FIG. 6B**) and a sequence encoding hANGPT1 (**FIG. 6C**) to induce overexpression of AOAH and ANGPT1. The effects of these MSCs overexpressing AOAH and ANGPT1 (NT003) was assessed in the pathogenic *E. coli*-induced model of sepsis described above, as well as in the cecal ligation and puncture (CLP)-induced sepsis model (**FIG. 7A**). Mice were treated with NT003 or vehicle 6h post-CLP, and survival was measured 5 days post-CLP. The results shown in **FIG. 6D** show that mice treated with NT003, but not with vehicle, were protected from death caused by pathogenic *E. coli*-induced sepsis. NT003 treatment was also shown to significantly improve survival in the CLP-induced sepsis model relative to vehicle, as shown in **FIG. 7B**.

## Claims

1. A mesenchymal stem cell (MSC) that is genetically modified to overexpress an acyloxyacyl hydrolase (AOAH) polypeptide.

2. The MSC of claim 1, wherein the MSC expresses a recombinant AOAH polypeptide.

3. The MSC of claim 2, wherein the recombinant AOAH polypeptide comprises an amino acid sequence that is at least 70% identical to the amino acid sequence of residues 35 to 575 of SEQ ID NO: 5.

4. The MSC of claim 3, wherein the recombinant AOAH polypeptide comprises the amino acid sequence of residues 35 to 575 of SEQ ID NO: 5, preferably wherein the recombinant AOAH polypeptide comprises the amino acid sequence of residues 24 to 575 of SEQ ID NO: 5.

5. The MSC of any one of claims 2 to 4, wherein the MSC comprises a first exogenous nucleic acid comprising an AOAH polypeptide-encoding region operably linked to a promoter or promoter/enhancer combination, preferably wherein the promoter is a cytomegalovirus (CMV) or elongation factor-1 (EF1) promoter or preferably wherein the promoter/enhancer combination is a CMV promoter/enhancer combination.

6. The MSC of claim 5, wherein the AOAH polypeptide-encoding region comprises a nucleotide sequence having at least 70% identity with nucleotides 7 to 1734 of SEQ ID NO:1, preferably comprises the nucleotide sequence of nucleotides 7 to 1734 of SEQ ID NO:1.

7. The MSC of any one of claims 1 to 6, wherein the MSC is genetically modified to overexpress an angiopoeitin-1 (ANGPT1) polypeptide, preferably wherein the MSC expresses a recombinant ANGPT1 polypeptide.

8. The MSC of claim 7, wherein the recombinant ANGPT1 polypeptide comprises an amino acid sequence that is at least 70% identical to the amino acid sequence of residues 16 to 498 of SEQ ID NO: 7, preferably comprises the amino acid sequence of residues 16 to 498 of SEQ ID NO: 7.

9. The MSC of any one of claims 7 or 8, wherein the MSC comprises a second exogenous nucleic acid comprising an ANGPT1 polypeptide-encoding region operably linked to a second promoter or promoter/enhancer combination.

10. The MSC of claim 9, wherein the second promoter is a CMV or EF1 promoter or wherein the second promoter/enhancer combination is a CMV promoter/enhancer combination.

11. The MSC of any one of claims 9 or 10, wherein the ANGPT1 polypeptide-encoding region comprises a nucleotide sequence having at least 70% identity with nucleotides 7 to 1503 of SEQ ID NO:3, preferably comprises the nucleotide sequence of nucleotides 7 to 1503 of SEQ ID NO:3.

12. The MSC of any one of claims 5 to 11, wherein the first and/or second exogenous nucleic acid(s) is/are comprised in a vector or plasmid.

13. The MSC of any one of claims 1 to 12, for use as a medicament.

14. The MSC of any one of claims 1 to 12 for use for the prevention or treatment of systemic inflammatory response syndrome (SIRS) in a subject, preferably wherein the SIRS is caused by sepsis and/or preferably wherein the subject is human.

15. The MSC for use according to claim 14, wherein the MSC are allogenic with respect to the subject.

## Patentansprüche

1. Mesenchymale Stammzelle (MSC), die genetisch modifiziert ist, um ein Polypeptid Acyloxyacylhydrolase (AOAH) überzuexprimieren.

2. MSC nach Anspruch 1, wobei die MSC ein rekombinantes AOAH-Polypeptid exprimiert.

3. MSC nach Anspruch 2, wobei das rekombinante AOAH-Polypeptid eine Aminosäuresequenz umfasst, die zu mindestens 70 % identisch mit der Aminosäuresequenz der Reste 35 bis 575 von SEQ ID NO: 5 ist.

4. MSC nach Anspruch 3, wobei das rekombinante AOAH-Polypeptid die Aminosäuresequenz der Reste 35 bis 575 von SEQ ID NO: 5 umfasst, vorzugsweise wobei das rekombinante AOAH-Polypeptid die Aminosäuresequenz der Reste 24 bis 575 von SEQ ID NO: 5 umfasst.

5. MSC nach einem der Ansprüche 2 bis 4, wobei die MSC eine erste exogene Nukleinsäure umfasst, umfassend eine für AOAH-Polypeptid kodierende Region, die funktionsfähig mit einem Promotor oder einer Promotor/Enhancer-Kombination verbunden ist, wobei der Promotor vorzugsweise ein Promotor von Cytomegalovirus (CMV) oder Elongationsfaktor-1 (EF1) ist, oder wobei die Promotor/Enhancer-Kombination vorzugsweise eine CMV-Promotor/Enhancer-Kombination ist.

6. MSC nach Anspruch 5, wobei die für AOAH-Polypeptid kodierende Region eine Nukleotidsequenz umfasst, die mindestens 70 % Identität mit den Nukleotiden 7 bis 1734 von SEQ ID NO: 1 aufweist, vorzugsweise die Nukleotidsequenz der Nukleotide 7 bis 1734 von SEQ ID NO: 1 umfasst.

7. MSC nach einem der Ansprüche 1 bis 6, wobei die MSC genetisch modifiziert ist, um ein Polypeptid Angiopoeitin-1 (ANGPT1) überzuexprimieren, vorzugsweise wobei die MSC ein rekombinantes ANGPT1-Polypeptid exprimiert.

8. MSC nach Anspruch 7, wobei das rekombinante ANGPT1-Polypeptid eine Aminosäuresequenz umfasst, die zu mindestens 70 % identisch mit der Aminosäuresequenz der Reste 16 bis 498 von SEQ ID NO: 7 ist, vorzugsweise die Aminosäuresequenz der Reste 16 bis 498 von SEQ ID NO: 7 umfasst.

9. MSC nach einem der Ansprüche 7 oder 8, wobei die MSC eine zweite exogene Nukleinsäure umfasst, die eine für ANGPT1-Polypeptid kodierende Region umfasst, die funktionsfähig mit einem zweiten Promotor oder einer Promotor/Enhancer-Kombination verbunden ist.

10. MSC nach Anspruch 9, wobei der zweite Promotor ein CMV- oder EF1-Promotor ist oder wobei die zweite Promotor/Enhancer-Kombination eine CMV-Promotor/Enhancer-Kombination ist.

11. MSC nach einem der Ansprüche 9 oder 10, wobei die für ANGPT1-Polypeptid kodierende Region eine Nukleotidsequenz mit mindestens 70 % Identität mit den Nukleotiden 7 bis 1503 von SEQ ID NO: 3 umfasst, vorzugsweise die Nukleotidsequenz der Nukleotide 7 bis 1503 von SEQ ID NO: 3 umfasst.

12. MSW nach einem der Ansprüche 5 bis 11, wobei die erste und/oder zweite exogene Nukleinsäure(n) in einem Vektor oder Plasmid enthalten ist/sind.

13. MSC nach einem der Ansprüche 1 bis 12 zur Verwendung als Medikament.

14. MSC nach einem der Ansprüche 1 bis 12 zur Verwendung zur Vorbeugung oder Behandlung von systemischen Entzündungsreaktionssyndrom (SIRS) bei einem Subjekt, wobei das SIRS vorzugsweise durch Sepsis verursacht wird und/oder vorzugsweise wobei das Subjekt ein Mensch ist.

15. MSC zur Verwendung nach Anspruch 14, wobei die MSC in Bezug auf das Subjekt allogen sind.

## Revendications

1. Cellule souche mésenchymateuse (CSM) génétiquement modifiée pour surexprimer un polypeptide d'hydrolase d'acyloxyacyle (AOAH).

2. CSM selon la revendication 1, dans laquelle la CSM exprime un polypeptide AOAH recombinant.

3. CSM selon la revendication 2, dans laquelle le polypeptide AOAH recombinant comprend une séquence d'acides aminés qui est au moins 70 % identique à la séquence d'acides aminés des résidus 35 à 575 de SEQ ID NO : 5.

4. CSM selon la revendication 3, dans lequel le polypeptide AOAH recombinant comprend la séquence d'acides aminés des résidus 35 à 575 de SEQ ID NO : 5, de préférence dans lequel le polypeptide AOAH recombinant comprend la séquence d'acides aminés des résidus 24 à 575 de SEQ ID NO : 5.

5. CSM selon l'une quelconque des revendications 2 à 4, dans laquelle la CSM comprend un premier acide nucléique exogène comprenant une région codant pour le polypeptide AOAH liée de manière opérationnelle à un promoteur ou à une combinaison promoteur/amplificateur, de préférence dans laquelle le promoteur est un promoteur du cytomégalovirus (CMV) ou du facteur d'élongation 1 (EF1), ou de préférence dans laquelle la combinaison promoteur/amplificateur est une combinaison promoteur/amplificateur du CMV.

6. CSM selon la revendication 5, dans laquelle la région codant pour le polypeptide AOAH comprend une séquence nucléotidique ayant au moins 70 % d'identité avec les nucléotides 7 à 1734 de SEQ ID NO : 1, et comprend de préférence la séquence nucléotidique des nucléotides 7 à 1734 de SEQ ID NO : 1.

7. CSM selon l'une quelconque des revendications 1 à 6, dans laquelle la CSM est génétiquement modifiée pour surexprimer un polypeptide d'angiopoïétine-1 (ANGPT1), de préférence dans laquelle la CSM exprime un polypeptide ANGPT1 recombinant.

8. CSM selon la revendication 7, dans laquelle le polypeptide ANGPT1 recombinant comprend une séquence d'acides aminés qui est au moins 70 % identique à la séquence d'acides aminés des résidus 16 à 498 de SEQ ID NO : 7, comprend de préférence la séquence d'acides aminés des résidus 16 à 498 de SEQ ID NO : 7.

9. CSM selon l'une quelconque des revendications 7 ou 8, dans laquelle la CSM comprend un second acide nucléique exogène comprenant une région codant pour le polypeptide ANGPT1 liée de manière opérationnelle à un second promoteur ou une seconde combinaison promoteur/amplificateur.

10. CSM selon la revendication 9, dans laquelle le second promoteur est un promoteur de CMV ou d'EF1 ou dans laquelle la seconde combinaison promoteur/amplificateur est une combinaison promoteur/amplificateur du CMV.

11. CSM selon l'une quelconque des revendications 9 ou 10, dans laquelle la région codant pour le polypeptide ANGPT1 comprend une séquence nucléotidique ayant au moins 70 % d'identité avec les nucléotides 7 à 1503 de SEQ ID NO : 3, et comprend de préférence la séquence nucléotidique des nucléotides 7 à 1503 de SEQ ID NO : 3.

12. CSM selon l'une quelconque des revendications 5 à 11, dans laquelle le premier et/ou le second acide nucléique exogène est/sont compris dans un vecteur ou un plasmide.

13. CSM selon l'une quelconque des revendications 1 à 12 à utiliser comme médicament.

14. CSM selon l'une quelconque des revendications 1 à 12 à utiliser pour la prévention ou le traitement du syndrome de réponse inflammatoire systémique (SIRS) chez un sujet, de préférence dans laquelle le SIRS est causé par une septicémie et/ou de préférence dans lequel le sujet est humain.

15. CSM à utiliser selon la revendication 14, dans laquelle les CSM sont allogènes par rapport au sujet.
